# EUROPEAN PATENT APPLICATION

(11) **EP 4 591 843 A1**
(43) Date of publication of application: **30.07.2025**
(21) Application number: 24153999.8
(22) Date of filing: 25.01.2024
(51) Int. Cl.: A61F 13/42

(54) **MOISTURE SENSING ARRANGEMENT, AND ABSORBENT ARTICLE COMPRISING MOISTURE SENSING ARRANGEMENT**

(71) Applicant: Mölnlycke Health Care AB, 431 21 Mölndal (SE)
(72) Inventor: REINHOLDSSON, Anders, 437 33 LINDOME (SE)
(74) Representative: Kransell & Wennborg KB

(57) **Abstract**

A moisture sensing arrangement (5) comprising a first sensing arrangement portion (9) having a first electrode (17) arranged therein, the first sensing arrangement portion (9) configured such that coverage of the first sensing arrangement (9) portion by an electrically conductive fluid (39) results in conductive contact between the first electrode (17) and the electrically conductive fluid (39); a second sensing arrangement portion (13) having a second electrode (19) arranged therein, the second sensing arrangement portion (13) being configured such that coverage of the second sensing arrangement portion (13) by an electrically conductive fluid (39) results in the second electrode (19) being separated from the electrically conductive fluid (39) by a dielectric layer (35); and capacitance estimation circuitry (21) coupled to the first electrode (17) and the second electrode (19), and being configured to estimate a capacitive coupling between the first electrode (17) and the second electrode (19).

## Description

### Field of the Invention

The present invention relates to a moisture sensing arrangement, and an absorbent article comprising such a moisture sensing arrangement.

### Background of the Invention

Absorbent articles intended for absorbing fluid from the body of a user may lose their ability to keep the skin of the user at a desired humidity level after having absorbed a certain amount of fluid. When conventional absorbent articles are used, it may therefore be necessary to perform regular manual checks, that may involve visual inspection, and may even need to involve to temporarily partly remove the absorbent article to be able to inspect the skin side of the absorbent article. The latter may particularly be the case if the absorbed fluid is substantially transparent. For absorbent articles in the form of wound dressings, it may be important for the healing process of the wound that the moisture level is kept below a certain limit, and that the wound dressing is replaced before its maximum capability of retaining fluid has been reached.

It would therefore be desirable to provide for improved moisture sensing in an absorbent article.

### Summary

It is an object of the present invention to provide for improved moisture sensing in an absorbent article.

According to an aspect of the present invention, it is therefore provided a moisture sensing arrangement for sensing moisture in an absorbent article, the moisture sensing arrangement comprising: a first sensing arrangement portion having a first electrode arranged therein, the first sensing arrangement portion being configured in such a way that coverage of the first sensing arrangement portion by an electrically conductive fluid results in electrically conductive contact between the first electrode and the electrically conductive fluid; a second sensing arrangement portion having a second electrode arranged therein, the second sensing arrangement portion being configured in such a way that coverage of the second sensing arrangement portion by an electrically conductive fluid results in the second electrode being separated from the electrically conductive fluid by a dielectric layer; and capacitance estimation circuitry coupled to the first electrode and the second electrode, the capacitance estimation circuitry being configured to estimate a capacitive coupling between the first electrode and the second electrode.

The present inventor has realized that existing methods for moisture sensing may not be optimal for use in an absorbent article. Resistive sensing requires the use of conductive measurement electrodes, which may experience problems with corrosion. Alternatively, it may be necessary to use expensive noble metals, which may not be suitable for a disposable product. Conventional capacitive sensing uses electrically insulated measurement electrodes, and therefore does not suffer from the problem of corrosion of the measurement electrodes. To be able to obtain reliable measurement values indicative of the moisture content in an absorbent article, the measurement electrodes may, however, need to be relatively large. This may reduce the number of absorbent articles for which conventional capacitive sensing is viable. Furthermore, large size measurement electrodes may make a conventional capacitive moisture sensing arrangement too costly for use in many types of disposable absorbent articles.

The present invention is based on the realization that a less costly and more compact capacitive moisture sensing arrangement for use in an absorbent article can be achieved by evaluating a capacitive coupling between a first electrode in conductive contact with electrically conductive fluid absorbed by the absorbent article and a second electrode that is separated from the absorbed fluid by a dielectric layer. With this configuration, the absorbed fluid can act as one of the plates in a parallel plate capacitor. This provides for a relatively large plate area (corresponding to a surface overlap between the absorbed fluid and the second electrode) and a relatively short distance between the plates (corresponding to the thickness of the dielectric layer separating the second electrode and the absorbed fluid), which results in a relatively high capacitance, when there is absorbed electrically conductive fluid that is in contact with the first electrode and overlapping with the second electrode. This provides for a strong dependence between absorbed fluid area coverage and capacitive coupling between the first electrode and the second electrode.

Another realization by the inventor is that the first electrode in the moisture sensing arrangement according to various examples of the present invention will not be nearly as sensitive to corrosion as in the case of resistive moisture sensing, because substantially no current will flow through the interface between the first electrode and the absorbed fluid.

Aspects of the present invention thus provide for improved moisture sensing in an absorbent article. For instance, moisture sensing can be achieved in a more cost-efficient and environmentally conscious manner.

The first electrode arranged in the first sensing arrangement portion may have an exposed conductive surface. The exposed conductive surface may be at least partly formed by a biocompatible electrically conductive material. This may be particularly relevant for absorbent articles intended to cover wounds, i.e., wound dressings. For absorbent articles in the form of hygiene articles, biocompatibility may not be needed.

Examples of suitable biocompatible electrically conductive materials may include silver/silver chloride, iridium oxide, boron/boron-doped diamond, a noble metal, and an oxide of a noble metal.

In an example configuration of the moisture sensing arrangement, the second sensing arrangement portion may, partly or completely, surround the first sensing arrangement portion. Such a configuration may be advantageous for making the first electrode relatively small and the second electrode relatively large, while still allowing the moisture sensing arrangement to be sensitive to relatively localized moisture in an absorbent article comprising the moisture sensing arrangement according to this example configuration.

The second electrode of the second sensing arrangement portion may be arranged on at least two substantially opposite sides of the first electrode of the first sensing arrangement portion. This configuration may allow the moisture sensing arrangement to sense the presence of absorbed fluid expanding between the first electrode and each of the two substantially opposite sides of the first electrode.

The second electrode of the second sensing arrangement portion may have a geometric center of mass overlapping with the first electrode of the first sensing arrangement portion. This may be beneficial for the ability to symmetrically sense the presence of absorbed fluid in different directions in relation to the first sensing arrangement portion of the moisture sensing arrangement. For instance, the moisture sensing arrangement may be arranged in an absorbent article in such a way that the first sensing arrangement portion is centered in a region of interest of the absorbent article. This may, for example, be a geometric center of mass of a wound dressing.

According to an example configuration of the moisture sensing arrangement of the present invention, a surface area of the second electrode of the second sensing arrangement portion may be at least two times a surface area of the first electrode of the first sensing arrangement. Increasing the area of the second electrode, both in absolute terms and in relation to the area of the first electrode may provide for improved sensitivity and/or precision in the moisture sensing.

Advantageously, the surface area of the second electrode of the second sensing arrangement portion may thus be at least five times the surface area of the first electrode of the first sensing arrangement.

According to an example configuration of the moisture sensing arrangement of the present invention, a longest distance between a point on a periphery of the first electrode of the first sensing arrangement portion and a point on a periphery of the second electrode of the second sensing arrangement portion may be at least 1.25 times longer than a shortest distance between a point on the periphery of the first electrode of the first sensing arrangement portion and a point on the periphery of the second electrode of the second sensing arrangement portion. This configuration provides for a gradually increasing capacitive coupling for an increasing surface coverage of the absorbed fluid in the absorbent article. The larger proportion of the second electrode that is overlapped by the absorbed fluid, the larger the capacitive coupling becomes, as long as the first electrode is also in electrically conductive contact with the electrically conductive absorbed fluid.

To ensure that an increase in surface coverage of absorbed fluid in a given direction in an absorbent article can also result in an increase in capacitive coupling between the first electrode and the second electrode of the moisture sensing arrangement, a longest distance along a line extending in a first direction between a point on the periphery of the first electrode of the first sensing arrangement portion and a point on the periphery of the second electrode of the second sensing arrangement portion may be at least 1.25 times longer than a shortest distance along a line extending in the first direction between a point on the periphery of the first electrode of the first sensing arrangement portion and a point on the periphery of the second electrode of the second sensing arrangement portion.

According to an example configuration of the moisture sensing arrangement of the present invention, the second electrode of the second sensing arrangement portion may comprise an oblong first sub-portion extending in a first radial direction in relation to a geometric center of mass of the first electrode of the first sensing arrangement portion; and an oblong second sub-portion extending in a second radial direction, different from the first radial direction, in relation to the geometric center of mass of the first electrode of the first sensing arrangement portion. This configuration provides for a gradually increasing capacitive coupling for an increasing surface coverage of the absorbed fluid in the absorbent article.

According to an example configuration of the moisture sensing arrangement of the present invention, the second sensing arrangement portion may have a third electrode, electrically insulated from the second electrode, arranged therein, the second sensing arrangement portion being configured in such a way that coverage of the second sensing arrangement portion by an electrically conductive fluid results in the third electrode being separated from the electrically conductive fluid by a dielectric layer; and the capacitance estimation circuitry may additionally be coupled to the third electrode and configured to estimate a first capacitive coupling between the first electrode and the second electrode and a second capacitive coupling between the first electrode and the third electrode. Adding a third electrode may provide for increase precision and sensitivity of the moisture sensing.

The moisture sensing arrangement according to various example configurations of the present invention may be included in an absorbent article, further comprising a fluid absorbing pad configured to absorb fluid from a body of a user. The moisture sensing arrangement may be arranged in contact with the fluid absorbing pad, in such a way that the first sensing arrangement portion and the second sensing arrangement portion of the moisture sensing arrangement can be covered by fluid absorbed by the fluid absorbing pad.

According to an example, the absorbent article may comprise a body contacting layer and a backing layer sandwiching the fluid absorbing pad; the fluid absorbing pad may be a layered structure comprising at least a fluid retention layer and a fluid absorption layer arranged between the fluid retention layer and the body contacting layer; and the moisture sensing arrangement may be arranged between the fluid retention layer and the fluid absorption layer.

### Brief Description of the Drawings

These and other aspects of the present invention will now be described in more detail, with reference to the appended drawings showing example embodiments of the invention, wherein:
Fig 1 is an illustration of an exemplary absorbent article, in the form of a wound dressing, arranged on the arm of a user;
Fig 2 is an exploded view of the absorbent article in fig 1, with an embedded moisture sensing arrangement according to an example embodiment of the present invention;
Figs 3A-C are cross-section views of the absorbent article in fig 2 with different amounts of absorbed fluid;
Fig 4 is a top-view of a moisture sensing arrangement according to a first embodiment of the invention;
Fig 5 is a top-view of a moisture sensing arrangement according to a second embodiment of the invention; and
Fig 6 is a top-view of a moisture sensing arrangement according to a third embodiment of the invention.

### Detailed Description of Example Embodiments

The present disclosure will now be described more fully hereinafter with reference to the accompanying drawings, in which example embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

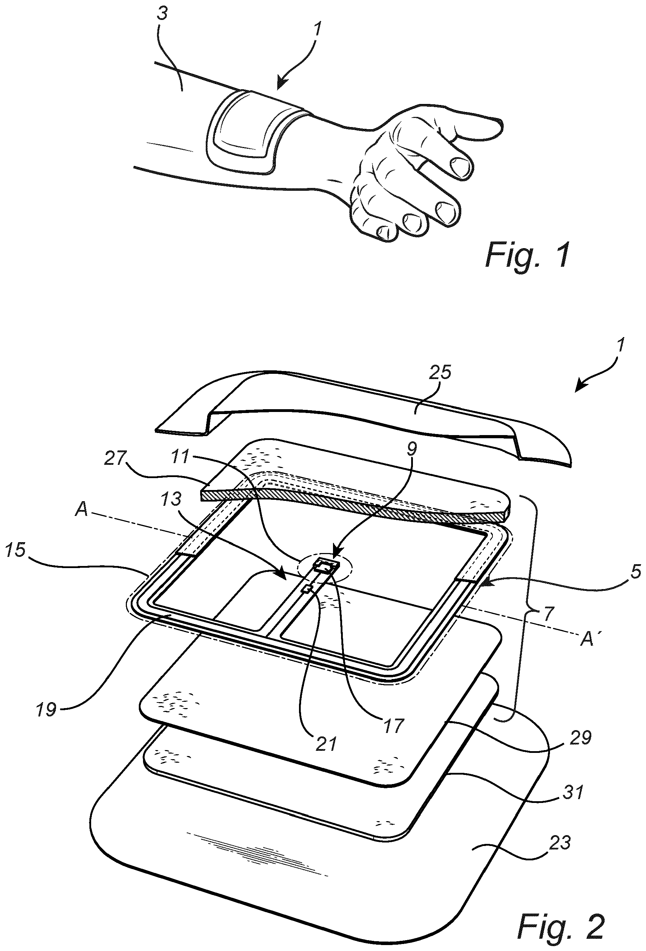

Fig 1 is an illustration of an exemplary absorbent article 1, in the form of a wound dressing, arranged on the arm 3 of a user. A wound dressing, such as that schematically shown in fig 1 may be a particularly interesting application for the moisture sensing arrangement according to embodiments of the present invention, because of the importance of the moisture level in the wound dressing for the healing of the wound.

Fig 2 is an exploded view of the absorbent article 1 in fig 1, with an embedded moisture sensing arrangement 5 according to an example embodiment of the present invention. The absorbent article (wound dressing) 1 in fig 2 comprises a fluid absorbing pad 7 configured to absorb fluid from the body of the user. In this particular case, the fluid may be exudate from a wound covered by the wound dressing. For other kinds of absorbent articles, the fluid may be urine or blood.

The moisture sensing arrangement 5 comprises a first sensing arrangement portion 9, schematically indicated by the inner dash-dot line 11 in fig 2, and a second sensing arrangement portion 13, between the inner dash-dot line 11 and the outer dash-dot line 15. The first sensing arrangement portion 9 has a first electrode 17 arranged therein, and the second sensing arrangement portion 13 has a second electrode 19 arranged therein.

The first sensing arrangement portion 9 is configured in such a way that coverage of the first sensing arrangement portion 9 by an electrically conductive fluid results in conductive contact between the first electrode 17 and the electrically conductive fluid, and the second sensing arrangement portion 13 is configured in such a way that coverage of the second sensing arrangement portion 13 by an electrically conductive fluid results in the second electrode 19 being separated from the electrically conductive fluid by a dielectric layer. The dielectric layer may be included in the moisture sensing arrangement 5, as will be exemplified below, or may be a layer of the absorbent article 1.

As is schematically indicated in fig 2, the moisture sensing arrangement 5 further comprises capacitance estimation circuitry 21 coupled to the first electrode 17 and the second electrode 19, and configured to estimate a capacitive coupling between the first electrode 17 and the second electrode 19. Various ways of estimating the capacitive coupling between two electrodes separated by a dielectric layer are, *per* se, well-known in the art, for instance from various types of capacitive sensors, capacitive touch screens, and capacitive fingerprint sensors, etc. Since it is considered to be straight-forward for the person of ordinary skill in the art to implement a suitable capacitive sensing technique in the moisture sensing arrangement according to various embodiments of the present invention, based on the information in the present application, no detailed information about techniques for measurement of capacitive coupling is provided herein.

In this example configuration, the absorbent article 1 comprises a body contacting layer 23 and a backing layer 25 sandwiching the fluid absorbing pad 7. The fluid absorbing pad 7 is, in this example configuration, a layered structure comprising a fluid retention layer 27, a fluid distribution layer 29, and a fluid absorption layer 31. The fluid absorption layer 31 is arranged closest to the body contacting layer 23, the fluid distribution layer 29 is arranged on the fluid absorption layer 31, and the fluid retention layer 27 is arranged on the fluid distribution layer 29. The moisture sensing arrangement 5 may advantageously be arranged between the fluid retention layer 27 and the fluid absorption layer 31. In particular, the moisture sensing arrangement 5 may be arranged between the fluid retention layer 27 and the fluid distribution layer 29.

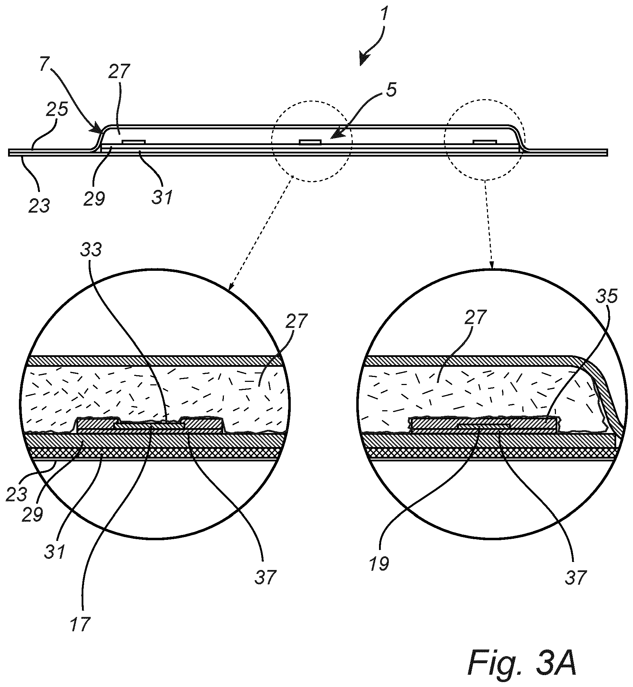

The functionality of the moisture sensing arrangement 5 according to an example embodiment of the present invention will now be described with reference to figs 3A-C, which are cross-section views of the absorbent article 1 in fig 2, of a section taken along the line A-A' in fig 2, with different amounts of fluid absorbed by the fluid absorbing pad 7 of the absorbent article 1.

As can be seen in the enlarged portions of fig 3A, the first electrode 17 has an exposed conductive surface 33, and the second electrode 19 is covered by a dielectric layer 35, which is here included in the moisture sensing arrangement 5. The first electrode 17 and the second electrode 19 may be included in a conductive layer formed on a flexible printed circuit board substrate 37. In that case, the conductive layer may, for example, be made of copper. The exposed conductive surface 33 may be formed by a biocompatible conductive material, suitable for use in an electrode. Examples of such materials include silver/silver chloride, iridium oxide, boron/boron-doped diamond, a noble metal, and an oxide of a noble metal. For this application, silver/silver chloride may be preferable, mainly for cost reasons. It should be noted that the listed materials are merely examples, and that other options exist and/or may be developed. Such additional examples include titanium and titanium nitride, various carbon-based materials, such as carbon nanotubes and graphene, and conductive polymers, such as PEDOT.

In fig 3A, the absorbent article 1 has not absorbed any fluid. There is a capacitive coupling C₀ between the first electrode 17 and the second electrode 19, but this capacitive coupling C₀ is very weak, since the electrode area is small, and the distance between the electrodes is large. This empty state capacitive coupling may be considered to be zero, for the purposes of estimating the amount and/or spatial distribution of fluid in the absorbent article 1.

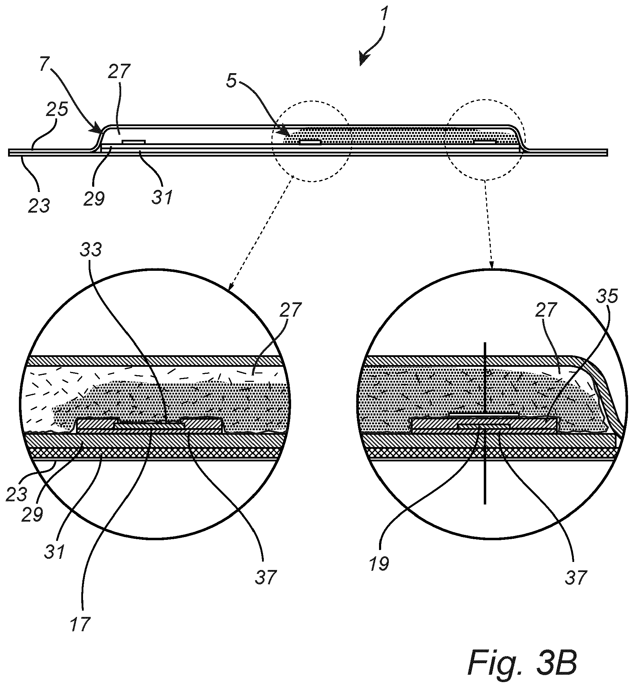

In fig 3B, the absorbent article 1 has absorbed some fluid 39, such as exudate, which mainly contains water and thus is electrically conductive. In the example state in fig 3B, the fluid 39 is in conductive contact with the first electrode 17, and covers the dielectric layer 35 on a portion of the second electrode 19, here to the right in fig 3B. As a result, a parallel plate capacitor - schematically indicated in the enlarged portion to the right in fig 3B - is formed by the fluid 39, which is conductively connected to the first electrode 17, and the portion of the second electrode 19 covered by the fluid 39. This configuration results in a capacitive coupling Cₐ between the first electrode 17 and the second electrode 19, which is proportional to area of the second electrode 19 that is covered by the fluid 39. Of course, this capacitive coupling Cₐ is much stronger than the capacitive coupling C₀ for the dry state shown in fig 3A.

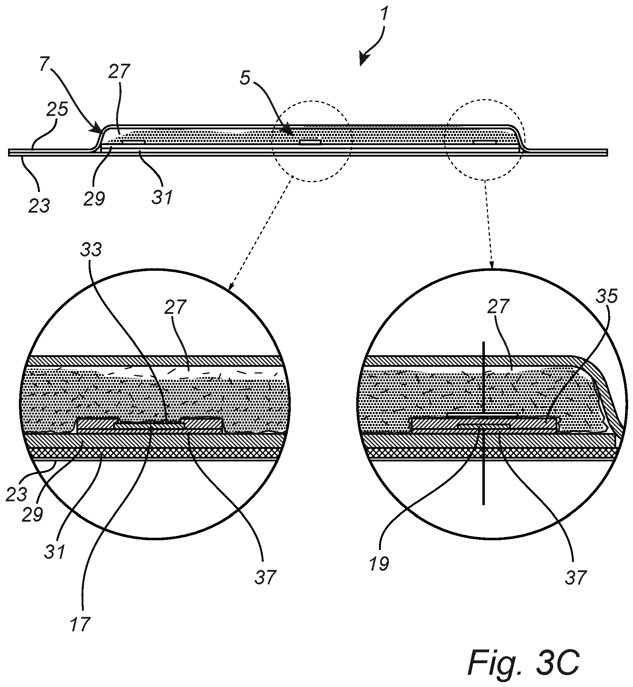

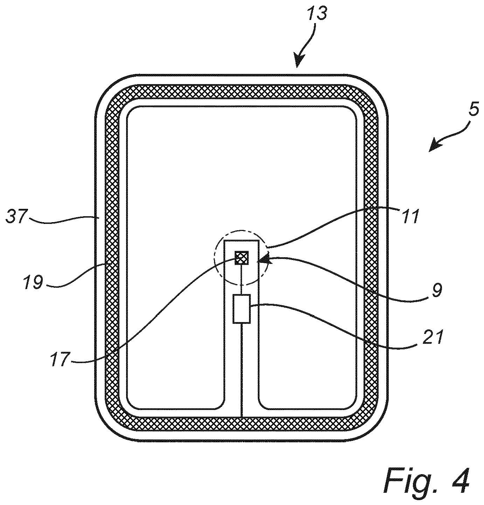

In fig 3C, the absorbent article 1 has absorbed more fluid 39 than in fig 3B. In the example state in fig 3C, the fluid 39 is in conductive contact with the first electrode 17, and covers the dielectric layer 35 on a larger portion of the second electrode 19, here to the right and left in fig 3C. This configuration results in a capacitive coupling C_{b} between the first electrode 17 and the second electrode 19, which is proportional to area of the second electrode 19 that is covered by the fluid 39. This capacitive coupling C_{b} is stronger than the capacitive coupling Cₐ for the state shown in fig 3B. As will be discussed below with reference to figs 4 to fig 6, different configurations of the moisture sensing arrangement 5 can provide different relations between spatial coverage of the fluid 39 in the absorbent article and the resulting capacitive coupling.

Fig 4 is a top-view of a moisture sensing arrangement 5 according to a first embodiment of the invention, with substantially the same configuration as that indicated as being included in the absorbent article 1 shown in fig 2 and figs 3A-C referred to above. In the first example configuration in fig 4, the second sensing arrangement portion 13 surrounds the first sensing arrangement portion 9. Furthermore, the second electrode 19 of the second sensing arrangement portion 13 is arranged on at least two substantially opposite sides of the first electrode 17 of the first sensing arrangement portion 9. Actually, in this example configuration, the second electrode 19 is arranged on all sides of the first electrode 17, so that presence of fluid can be sensed on all sides of the first electrode 17, as long as the first electrode 17 is covered by the fluid. It can also be easily understood from fig 4, that the second electrode 19 has a geometric center of mass which overlaps with the first electrode 17. This configuration contributes to symmetry of sensing in relation to the first electrode 17. Moreover, the area of the second electrode 19 is clearly much larger than the area of the first electrode 17. The first electrode 17 and the second electrode 19 are on a flexible substrate 37. As is shown in fig 4, and as was also shown in the cross-section views in figs 3A-C, the flexible substrate 37 is only partly present between the first electrode 17 and the second electrode 19, in this configuration only where the capacitance estimation circuitry 21 is arranged, and where the first electrode 17 and the second electrode 19 are connected to the capacitance estimation circuitry 21. The moisture sensing arrangement 5 may be arranged in the absorbent article facing up, as indicated in figs 3A-C, or facing down. The moisture sensing arrangement 5 according to the different embodiments of the present invention will not indicate a strong capacitive coupling between the first electrode 17 and the second electrode 19 unless the absorbed fluid at least partly overlaps with both the first electrode 17 and the second electrode 19. The first embodiment of the invention shown in fig 4 has a simple configuration, but requires a relatively large area of the moisture sensing arrangement 5 to be overlapped by absorbed fluid before it can be estimated that the absorbent article contains absorbed fluid. This may be sufficient for some applications. In other applications, it may be desirable to be able to detect when a smaller area has been covered by absorbed fluid and/or to provide an indication of the size and/or spatial distribution of the absorbed fluid.

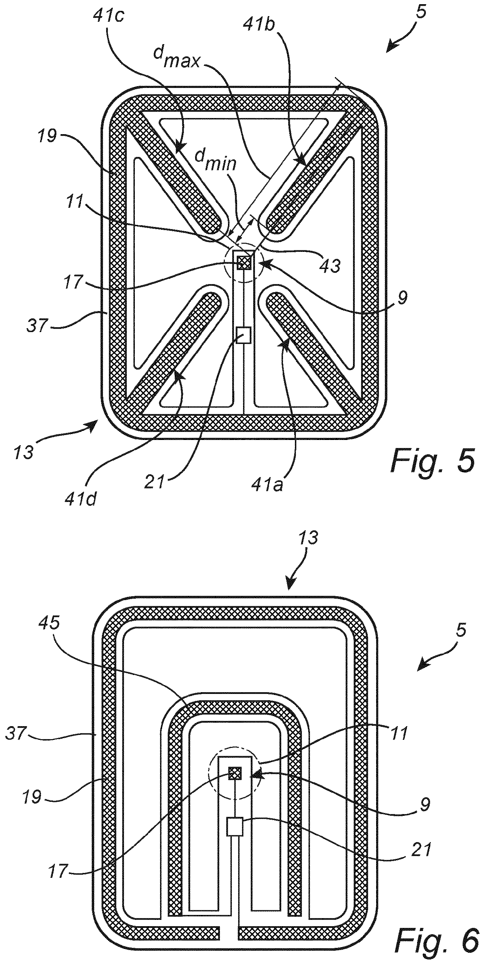

Fig 5 is a top-view of a moisture sensing arrangement 5 according to a second embodiment of the invention. The moisture sensing arrangement 5 in fig 5 can be seen as a modification of the configuration in fig 4, where the second electrode 19 of the second sensing arrangement portion 13 comprises oblong sub-portions 41a-d extending in different radial directions in relation to a geometric center of mass of the first electrode 17. With this configuration of the second electrode 19, a gradually increasing surface coverage of absorbed fluid in the absorbent article 1 results in a gradually increasing capacitive coupling between the first electrode 17 and the second electrode 19, which may be beneficial for certain types of absorbent articles, such as wound dressings, at least for certain applications. The example configuration of the moisture sensing arrangement 5 in fig 5 also exhibits a longest distance dₘₐₓ along a line 43 extending in a first direction between a point on a periphery of the first electrode 17 and a first point on a periphery of the second electrode 19 that is more than 1.25 times longer than a shortest distance dₘᵢₙ along the line 43 extending in the first direction between a point on the periphery of the first electrode 17 and a second point on the periphery of the second electrode 19.

Fig 6 is a top-view of a moisture sensing arrangement 5 according to a third embodiment of the invention. The moisture sensing arrangement 5 in fig 6 can be seen as a modification of the configuration in fig 4, where the second sensing arrangement portion 13 has a third electrode 45, electrically insulated from the second electrode 19 and covered by a dielectric layer. As is schematically indicated in fig 6, the capacitance estimation circuitry 21 is additionally coupled to the third electrode 45. The capacitance estimation circuitry 21 is configured to estimate a first capacitive coupling between the first electrode 17 and the second electrode 19 and a second capacitive coupling between the first electrode 17 and the third electrode 45. This provides for improved precision in the estimation of the amount and/or spatial coverage of absorbed fluid in the absorbent article 1 comprising the moisture sensing arrangement 5.

In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage.

## Claims

1. A moisture sensing arrangement (5) for sensing moisture in an absorbent article (1), the moisture sensing arrangement (5) comprising:
a first sensing arrangement portion (9) having a first electrode (17) arranged therein, the first sensing arrangement portion (9) being configured in such a way that coverage of the first sensing arrangement (9) portion by an electrically conductive fluid (39) results in conductive contact between the first electrode (17) and the electrically conductive fluid (39);
a second sensing arrangement portion (13) having a second electrode (19) arranged therein, the second sensing arrangement portion (13) being configured in such a way that coverage of the second sensing arrangement portion (13) by an electrically conductive fluid (39) results in the second electrode (19) being separated from the electrically conductive fluid (39) by a dielectric layer (35); and
capacitance estimation circuitry (21) coupled to the first electrode (17) and the second electrode (19), the capacitance estimation circuitry (21) being configured to estimate a capacitive coupling between the first electrode (17) and the second electrode (19).

2. The moisture sensing arrangement (5) according to claim 1, the first electrode (17) arranged in the first sensing arrangement portion (9) having an exposed conductive surface (33).

3. The moisture sensing arrangement (5) according to claim 2, the exposed conductive surface (33) being at least partly formed by a biocompatible electrically conductive material.

4. The moisture sensing arrangement (5) according to claim 3, the biocompatible electrically conductive material being silver/silver chloride or iridium oxide or boron/boron-doped diamond, or a noble metal, or an oxide of a noble metal.

5. The moisture sensing arrangement (5) according to any one of the preceding claims, the second sensing arrangement portion (13) surrounding the first sensing arrangement portion (9).

6. The moisture sensing arrangement (5) according to claim 5, the second electrode (19) of the second sensing arrangement portion (13) being arranged on at least two substantially opposite sides of the first electrode (17) of the first sensing arrangement portion (9).

7. The moisture sensing arrangement (5) according to claim 6, the second electrode (19) of the second sensing arrangement portion (13) having a center of mass overlapping with the first electrode (17) of the first sensing arrangement portion (9).

8. The moisture sensing arrangement (5) according to any one of the preceding claims, a surface area of the second electrode (19) of the second sensing arrangement portion (13) being at least two times a surface area of the first electrode (17) of the first sensing arrangement portion (9).

9. The moisture sensing arrangement (5) according to claim 8, the surface area of the second electrode (19) of the second sensing arrangement portion (13) being at least five times the surface area of the first electrode (17) of the first sensing arrangement portion (9).

10. The moisture sensing arrangement (5) according to any one of the preceding claims, a longest distance (dₘₐₓ) between a point on a periphery of the first electrode (17) of the first sensing arrangement portion (9) and a point on a periphery of the second electrode (19) of the second sensing arrangement portion (13) being at least 1.25 times longer than a shortest distance (dₘᵢₙ) between a point on the periphery of the first electrode (17) of the first sensing arrangement portion (13) and a point on the periphery of the second electrode (19) of the second sensing arrangement portion (13).

11. The moisture sensing arrangement (5) according to claim 10, a longest distance (dₘₐₓ) along a line (43) extending in a first direction between a point on the periphery of the first electrode (17) of the first sensing arrangement portion (9) and a point on the periphery of the second electrode (19) of the second sensing arrangement portion (13) being at least 1.25 times longer than a shortest distance (dₘᵢₙ) along a line extending in the first direction between a point on the periphery of the first electrode (17) of the first sensing arrangement portion (9) and a point on the periphery of the second electrode (19) of the second sensing arrangement portion (13).

12. The moisture sensing arrangement (5) according to any one of the preceding claims, the second electrode (19) of the second sensing arrangement portion (13) comprising:
an oblong first sub-portion (41a) extending in a first radial direction in relation to a center of mass of the first electrode (17) of the first sensing arrangement portion (9); and
an oblong second sub-portion (41b) extending in a second radial direction, different from the first radial direction, in relation to the center of mass of the first electrode (17) of the first sensing arrangement portion (9).

13. The moisture sensing arrangement (5) according to any one of the preceding claims:
the second sensing arrangement portion (13) having a third electrode (45), electrically insulated from the second electrode (19), arranged therein, the second sensing arrangement portion (13) being configured in such a way that coverage of the second sensing arrangement portion (13) by an electrically conductive fluid (39) results in the third electrode (45) being separated from the electrically conductive fluid (39) by a dielectric layer (35); and
the capacitance estimation circuitry (21) additionally being coupled to the third electrode (45), the capacitance estimation circuitry (21) being configured to estimate a first capacitive coupling between the first electrode (17) and the second electrode (19) and a second capacitive coupling between the first electrode (17) and the third electrode (45).

14. An absorbent article (1) comprising:
a fluid absorbing pad (7) configured to absorb fluid (39) from a body of a user; and
the moisture sensing arrangement (5) according to any one of the preceding claims arranged in contact with the fluid absorbing pad (7), in such a way that the first sensing arrangement portion (9) and the second sensing arrangement portion (13) of the moisture sensing arrangement (5) can be covered by fluid (39) absorbed by the fluid absorbing pad (7).

15. The absorbent article (1) according to claim 14, wherein:
the absorbent article (1) comprises a body contacting layer (23) and a backing layer (25) sandwiching the fluid absorbing pad (7);
the fluid absorbing pad (7) is a layered structure comprising at least a fluid retention layer (27) and a fluid absorption layer (31) arranged between the fluid retention layer (27) and the body contacting layer (23); and
the moisture sensing arrangement (5) is arranged between the fluid retention layer (27) and the fluid absorption layer (31).
